# EUROPÄISCHE PATENTSCHRIFT

(11) **EP 2 117 525 B1**
(45) Veröffentlichungstag und Bekanntmachung des Hinweises auf die Patenterteilung: **25.12.2013**
(21) Anmeldenummer: 08716394.5
(22) Anmeldetag: 10.03.2008
(51) Int. Cl.: A61K 31/137, A61P 29/00

(54) **VERWENDUNG VON 1-PHENYL-3-DIMETHYLAMINO-PROPANVERBINDUNGEN ZUR THERAPIE DES NEUROPATHIESCHMERZES**
USE OF 1-PHENYL-3-DIMETHYLAMINO-PROPANE COMPOUNDS FOR THE THERAPY OF NEUROPATHY PAIN
UTILISATION DE COMPOSÉS DE 1-PHÉNYL-3-DIMÉTHYLAMINO-PROPANE POUR TRAITER LA DOULEUR NEUROPATHIQUE

(30) Priorität: 12.03.2007 DE 102007012165
(43) Veröffentlichungstag der Anmeldung: 18.11.2009
(73) Patentinhaber: Grünenthal GmbH, 52078 Aachen (DE)
(72) Erfinder: CHRISTOPH, Thomas, 52080 Aachen (DE); KÖGEL, Babette-Yvonne, 52379 Langerwehe-Hamich (DE); FRIDERICHS, Elmar, 52223 Stolberg (DE); MEEN, Muriel, Dr., 31450 AYGUESVIVES (FR)
(86) Internationale Anmeldenummer: PCT/EP2008/001884
(87) Internationale Veröffentlichungsnummer: WO 2008/110323

(56) Entgegenhaltungen:
- WO-A-2004/047823
- WO-A-2007/128413
- TZSCHENTKE T M ET AL: "Tapentadol hydrochloride. Analgesic, Mu-opioid receptor agonist, noradrenaline reuptake inhibitor" DRUGS OF THE FUTURE, BARCELONA, ES, Bd. 31, Nr. 12, 1. Dezember 2006 (2006-12-01), Seiten 1053-1061, XP002438122 ISSN: 0377-8282

## Beschreibung

Die Erfindung betrifft die Verwendung von (1R,2R)-3-(3-Dimethylamino-1-ethyl-2-methylpropyl)-phenol zur Herstellung von Arzneimitteln zur Behandlung von diabetischem neuropathischem Schmerz oder diabetischerNeuropathie; bevorzugt diabetischem peripherem neuropathischem Schmerz, weiterhin bevorzugt zur Behandlung von diabetischer peripheraler Neuropathie.

Die normale physiologische Schmerzempfindung, die dem Organismus als Schutzfunktion zur Verfügung steht, wird über Nervenfasern durch entsprechende schmerzhafte Stimuli vermittelt. Man spricht von nozizeptivem Schmerz. Dieser nozizeptive Schmerz kann akut oder chronisch, somatisch oder viszeral, mit oder ohne Entzündungskomponente vorliegen. Adäquate Stimuli können mechanisch (z.B. Druck), thermisch (z.B. Hitze) oder chemisch (z.B. Säure) sein. Auch elektrische Reize können als schmerzhaft wahrgenommen werden.

Im Gegensatz zum nozizeptiven Schmerz - und auch meist nicht mit den gleichen Mitteln behandelbar- zeichnet sich der neuropathische Schmerz (ein nicht-nozizeptiver Schmerz; zur Übersicht siehe Hansson et al., 2001 Neuropathic Pain: Patophysiology and Treatment in Progress in Pain Research and Management, Vol.21 eds. Hansson et al. IASP Press, Seattle; Bridges et al., 2001 Br J Anaesthesia 87:12-26) durch das Auftreten spontaner und/oder durch abnormale Stimuli ausgelöste Schmerzen aus. Spontan auftretender Schmerz resultiert z.B. aus sogenannter ektopischer Aktivität der schmerzleitenden Nervenfasern. In diesem Fall sendet die Nervenfaser ein Schmerzsignal von der Peripherie ins ZNS, ohne daß ein adäquater Stimulus vorhanden wäre. Ein Beispiel für Schmerz, der durch einen abnormalen Stimulus ausgelöst wird, ist das Phänomen der Allodynie. Definiert ist die Allodynie als schmerzhafte Empfindung eines normalerweise nicht-schmerzhaften Stimulus. Eine Allodynie ist nicht auf neuropathischen Schmerz beschränkt. So tritt Allodynie z.B. in nicht-neuropathischen Bedingungen wie Sonnenbrand oder Gelenkentzündungen auf. Die zugrunde liegenden Mechanismen der Allodynie unterscheiden sich jedoch grundsätzlich voneinander und sind durch gründliche medizinische Anamnese und Untersuchung klassifizierbar.

Ein weiteres Beispiel für abnormale Schmerzempfindung ist die Hyperalgesie. In diesem Fall wird ein normalerweise schmerzhafter Stimulus als stärkerer Schmerz wahrgenommen als dies in einer gesunden Situation der Fall ist. Diese Art der verstärketen Schmerzwahrnehmung kommt nicht nur im Neuropathieschmerz sondern auch zum Beispiel im Entzündungsschmerz vor, hat dort aber eine andere Ursache (Entzündung) als im Neuropathieschmerz.

Veschiedene Stoffwechselerkrankungen können die Ursache zu neuropathischen Veränderungen sein und in der Folge mit neuropathischen Schmerzen einhergehen. Ein Beispiel für eine solche Neuropathie ist die diabetische Neuropathie welche bei einer großen Zahl von Patienten mit Diabetes mellitus auftritt und mit einer Vielzahl klinischer Symptome einhergehen kann wie zum Beispiel Taubheitsgefühle, Kribbeln oder Schmerzen. Die häufigste Form der diabetischen Neuropthie ist die distale symmetrische sensomotorische Polyneuropathie.

Neuropathischer Schmerz tritt u.a. nach Schädigung peripherer oder zentraler Nerven auf und lässt sich dementsprechend tierexperimentell durch gezielte Läsionen einzelner Nerven induzieren und beobachten. Zwei mögliche Tiermodelle sind die Nervenläsion nach Bennett (Bennett and Xie, 1988 Pain 33: 87-107) sowie die nach Chung (Kim and Chung, 1992 Pain 50:355-363). Im Bennett Modell wird der Ischiasnerv unilateral mit losen Ligaturen versehen; im Chung Modell werden unilateral zwei Spinalnerven abgebunden. In beiden Fällen ist die Entwicklung von Anzeichen des Neuropathieschmerz zu beobachten und kann mittels thermischer oder mechanischer Allodynie quantifiziert werden.

Ein bekanntes Tiermodell zur Untersuchung diabetischer Neuropathie ist die Induktion von Diabetes bei Nagern durch die einmalige Gabe von Streptozotozin, einem antibiotischen Extrakt aus Streptomyces acromogenes, welches selektiv die ß-Zellen des Pankras schädigt. Die Tiere zeigen nach einiger Zeit typische Symptome des diabetischen Neuropathieschmerzes wie zum Beispiel mechanische, thermische oder chemische Hyperalgesie (Courteix et al., 1993 Pain 53: 81-88).

T.M. Tzschenthe et al., Drügs of the Fütüre 2006, 31(12): 1053-1061 ofenbart, dass Tapentadol im Tiermodell analschiste Wirksamkeit u.a. gegen chronishen neüropathischen Schmerz Zeigt.

Zur Behandlung wird u.a. Gabapentin eingesetzt, das aber nur eine relativ geringe Wirksamkeit zeigt, bzw. erst bei erheblichen Dosierungen wirkt. Auf der anderen Seite wird häufig auch Morphin eingesetzt, dessen Nebenwirkungsprofil bekanntermaßen nicht unproblematisch ist. Angesichts dieses Standes der Technik bestand Bedarf nach Verbindungen mit einem günstigen Verhältnis zwischen Wirksamkeit und Nebenwirkung bzw. Verbindungen zur Behandlung von neuropathischem Schmerz.

Aufgabe der vorliegenden Erfindung war es daher, Verbindungen mit Wirksamkeit im neuropathischem, insbesondere polyneuropathischem und insbesondere diabetischem Schmerz aufzufinden. Das ist dadurch erschwert, daß ein großer bis überwiegender Teil der im nozizeptivem Schmerz - wie dem Akkutschmerz - wirksamen Substanzen im neuropathischen Schmerz gar nicht oder nur schwach wirksam sind.

Überraschenderweise wurde nun gefunden, daß (1R,2R)-3-(3-Dimethylamino-1-ethyl-2-methylpropyl)-phenol im diabetisch neuropathischen Schmerz hochgradig wirksam war.

Dementsprechend ist Erfindungsgegenstand die Verwendung von (1R,2R)-3-(3-Dimethylamino-1-ethyl-2-methylpropyl)-phenol in dargestellter Form oder in Form ihrer Säuren oder ihrer Basen oder in Form ihrer Salze, insbesondere der physiologisch verträglichen Salze, oder in Form ihrer Solvate, insbesondere der Hydrate;
zur Herstellung eines Arzneimittels zur Behandlung von diabetischen neuropathischem Schmerz, bevorzugt diabetischen peripheren neuropathischen Schmerz, oder von diabetischer Neuropathie und sehr bevorzugt diabetischer peripherer Neuropathie.

Etwa 1% der Bevölkerung litt unter neuropathischem insbesondere unter polyneuropathischem Schmerz, es ist eine der am schwierigsten zu behandelnden Schmerzarten. Es besteht Bedarf nach wirksamen Medikamenten zur Behandlung von insbesonderen diabetischen neuropathischen Schmerzen., insbesondere für Patienten die sensibel sind bezgl. Nebenwirkungen von NSAID Schmerzmitteln und auf dem Markt befindlichen µ-opioid Agonisten, Antdepressiva und Anticonvulsiva bzw. deren Schmerzen nicht ausreichend mit anderen nicht-Opiod Analgetika, Antidepressiva und Anticonvulsiva behandelt werden können.

Überraschenderweise hat sich herausgestellt, daß die erfindüngsgemäße Substanz hervorragende Wirksamkeit in den zwei wichtigsten In-vivo-Modellen des neuropathischen Schmerzes und was besonders überraschend und hervorzuheben ist insbesondere im in-vivo-Modell für diabetische Neuropathie zeigt. Die besondere Selektivitat hinsichtlich polyneuropathischem und diabetischem neuropathischem Schmerz wird im weiteren in-vivo-Modellen (Beispiel 6) gezeigt; belegt durch einen klaren Unterschied in der Wirkstärke (Faktor 3!). Im übrigen war Gabapentin erheblich schlechter wirksam als diese Verbindung.

Gegenstand der Erfindung ist die Verwendung der Substanz (1R,2R)-3-(3-Dimethylamino-1-ethyl-2-methyl-propyl)-phenol, ein zentral aktives Analgetikum, welches ein "dual mode of action" hat (µ-opioid Rezeptor Agonist und ein Inhibitor von Noradrenaline uptake) und verknüpft ist mit geringen Opioid-typischen Nebenwirkungen um Gegensatz zu aktuell gebräulichen und auf dem Markt befindlichen Opioden, zur Behandlung von diabetischen neuropathischen, bevorzugt diabetisch peripheren neuropathischen Schmerz.

Im Sinne dieser Erfindung versteht man unter Alkyl- bzw. Cykloalkyl-Resten gesättigte und ungesättigte (aber nicht aromatische), verzweigte, unverzweigte und cyclische Kohlenwasserstoffe, die unsubstituiert oder ein- oder mehrfach substituiert sein können. Dabei steht C₁₋₂-Alkyl für C1- oder C2-Alkyl, C₁₋₃-Alkyl für C1-, C2- oder C3-Alkyl, C₁₋₄-Alkyl für C1-, C2-, C3-oder C4-Alkyl, C₁₋₅-Alkyl für C1-, C2-, C3-, C4- oder C5-Alkyl, C₁₋₆-Alkyl für C1-, C2-, C3-, C4-, C5- oder C6-Alkyl, C₁₋₇-Alkyl für C1-, C2-, C3-, C4-, C5-, C6- oder C7-Alkyl, C₁₋₈-Alkyl für C1-, C2-, C3-, C4-, C5-, C6-, C7- oder C8-Alkyl, C₁₋₁₀-Alkyl für C1-, C2-, C3-, C4-, C5-, C6-, C7-, C8,- C9- oder C10-Alkyl und C₁₋₁₈-Alkyl für C1-, C2-, C3-, C4-, C5-, C6-, C7-, C8,- C9-, C10-, C11-, C12-, C13-, C14-, C15-, C16-, C17- oder C18-Alkyl. Weiter steht C₃₋₄-Cycloalkyl für C3- oder C4-Cycloalkyl, C₃₋₅-Cycloalkyl für C3-, C4- oder C5-Cycloalkyl, C₃₋₆-Cycloalkyl für C3-, C4-, C5- oder C6-Cycloalkyl, C₃₋₇-Cycloalkyl für C3-, C4-, C5-, C6- oder C7-Cycloalkyl, C₃₋₈-Cycloalkyl für C3-, C4-, C5-, C6-, C7- oder C8-Cycloalkyl, C₄₋₅-Cycloalkyl für C4- oder C5-Cycloalkyl, C₄₋₆-Cyctoalkyl für C4-, C5- oder C6-Cycloalkyl, C₄₋₇-Cycloalkyl für C4-, C5-, C6- oder C7-Cycloalkyl, C₅₋₆-Cycloalkyl für C5- oder C6-Cycloalkyl und C₅₋₇-Cycloalkyl für C5-, C6- oder C7-Cycloalkyl. In Bezug auf Cycloalkyl umfaßt der Begriff auch gesättigte Cycloalkyle, in denen ein oder 2 Kohlenstoffatome durch ein Heteroatom, S, N oder O ersetzt sind. Unter den Begriff Cycloalkyl fallen aber insbesondere auch ein- oder mehrfach, vorzugsweise einfach, ungesättigte Cycloalkyle ohne Heteroatom im Ring, solange das Cycloalkyl kein aromatisches System darstellt. Vorzugsweise sind die Alkyl- bzw. Cykloalkyl-Reste Methyl, Ethyl, Vinyl (Ethenyl), Propyl, Allyl (2-Propenyl), 1-Propinyl, Methylethyl, Butyl, 1-Methylpropyl, 2-Methylpropyl, 1,1-Dimethylethyl, Pentyl, 1,1-Dimethylpropyl, 1,2-Dimethylpropyl, 2,2-Dimethylpropyl, Hexyl, 1-Methylpentyl, Cyclopropyl, 2-Methylcyclopropyl, Cyclopropylmethyl, Cyclobutyl, Cyclopentyl, Cyclopentylmethyl, Cyclohexyl, Cycloheptyl, Cyclooctyl, aber auch Adamantyl, CHF₂, CF₃ oder CH₂OH sowie Pyrazolinon, Oxopyrazolinon, [1,4]Dioxan oder Dioxolan.

Dabei versteht man im Zusammenhang mit Alkyl und Cycloalkyl - solange dies nicht ausdrücklich anders definiert ist - unter dem Begriff substituiert im Sinne dieser Erfindung die Substitution mindestens eines (gegebenenfalls auch mehrerer) Wasserstoffreste(s) durch F, Cl. Br, I, NH₂, SH oder OH, wobei unter "mehrfach substituiert" bzw. "substituiert" bei mehrfacher Substitution zu verstehen ist, daß die Substitution sowohl an verschiedenen als auch an gleichen Atomen mehrfach mit den gleichen oder verschiedenen Substituenten erfolgt, beispielsweise dreifach am gleichen C-Atom wie im Falle von CF₃ oder an verschiedenen Stellen wie im Falle von -CH(OH)-CH=CH-CHCl₂. Besonders bevorzugte Substituenten sind hier F, Cl und OH. In Bezug auf Cycloalkyl kann der Wasserstoffrest auch durch OC₁₋₃-Alkyl oder C₁₋₃-Alkyl (jeweils ein- oder mehrfach substituiert oder unsubstituiert), insbesondere Methyl, Ethyl, n-Propyl, i-Propyl, CF₃, Methoxy oder Ethoxy, ersetzt sein.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen, unter (CH₂)₁₋₄ ist -CH₂-. -CH₂-CH₂-, -CH₂-CH₂-CH₂- und -CH₂-CH₂-CH₂-CH₂- zu verstehen, unter (CH₂)₄₋₅ ist -CH₂-CH₂-CH₂-CH₂- und -CH₂-CH₂-CH₂-CH₂-CH₂- zu verstehen, etc.

Unter einem Aryl-Rest werden Ringsysteme mit mindestens einem aromatischen Ring aber ohne Heteroatome in auch nur einem der Ringe verstanden. Beispiele sind Phenyl-, Naphthyl-, Fluoranthenyl-, Fluorenyl-, Tetralinyl- oder Indanyl, insbesondere 9H-Fluorenyl- oder Anthracenyl-Reste, die unsubstituiert oder einfach oder mehrfach substituiert sein können.

Unter einem Heteroaryl-Rest werden heterocyclische Ringsysteme mit mindestens einem ungesättigten Ring verstanden, die ein oder mehrere Heteroatome aus der Gruppe Stickstoff, Sauerstoff und/oder Schwefel enthalten und auch einfach oder mehrfach substituiert sein können. Beispielhaft seien aus der Gruppe der Heteroaryle Furan, Benzofuran, Thiophen, Benzothiophen, Pyrrol, Pyridin, Pyrimidin, Pyrazin, Chinolin, Isochinolin, Phthalazin, Benzo[1,2,5]thiadiazol, Benzothiazol, Indol, Benzotriazol, Benzodioxolan, Benzodioxan, Carbazol, Indol und Chinazolin aufgeführt.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter physiologisch verträglich ist zu verstehen, daß die Substanz, insbesondere das Salz als solches, bei Anwendung im Menschen oder Säugetier verträglich ist, also beispielsweise nicht unphysiologisch (z.B. giftig) wirkt.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch-insbesondere bei Anwendung im Menschen und/oder Säugetier-veträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, 1,1-Dioxo-1,2-dihydro1b6-benzo[d]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, a-Liponsäure, Acetylglycin, Acetylsalicylsäure, Hippursäure und/oder Asparaginsäure. Besonders bevorzugt ist das Hydrochlorid-Salz.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, 1,1-Dioxo-1,2-dihydro1λ⁶-benzo[*d*]isothiazol-3-on (Saccharinsäure), Monomethylsebacinsäure, 5-Oxo-prolin, Hexan-1-sulfonsäure, Nicotinsäure, 2-, 3- oder 4-Aminobenzoesäure, 2,4,6-Trimethylbenzoesäure, α-Liponsäure, Acetylglycin, Acetylsalicylsäure, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure-als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali-und Erdalkalimetalle aber auch mit NH₄⁺, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch NH₄⁺, insbesondere aber (Mono-) oder (Di-) Natrium-, (Mono-) oder (Di-) Kalium-, Magnesium- oder Calzium-Salze.

Die erfindungsgemäß verwendete Verbindung und deren Herstellung ist aus der DE 44 26 245 A1 prinzipiell bekannt.

Die Arzneimittel zur Behandlung von diabetischen neuropathischem Schmerz, bevorzugt diabetischen peripheren neuropathischen Schmerz, oder zur Behandlung von diabetischer Neuropathie, sehr bevorzugt diabetischer peripherer Neuropathie,
zu deren Herstellung die genannte Verbnindung erfindungsgemäß benutzt wird, enthalten mindestens den erfindungsgemäß verwendeten, genannten Wirkstoff sowie gegebenenfalls geeignete Zusatz- und/oder Hilfsstoffe.

Geeignete Zusatz- und/oder Hilfsstoffe im Sinne dieser Erfindung sind alle dem Fachmann aus dem Stand der Technik bekannten Stoffe zur Erreichung galenischer Formulierungen. Die Auswahl dieser Hilfsstoffe sowie die einzusetzenden Mengen derselben hängen davon ab, ob das Arzneimittel oral, intravenös, intraperitoneal, intradermal, intramuskulär, intranasal, buccal oder lokal appliziert werden soll. Für die orale Applikation eignen sich Zubereitungen in Form von Tabletten, Kautabletten, Dragees, Kapseln, Granulaten, Tropfen, Säften oder Sirupen, für die parenterale, topische und inhalative Applikation Lösungen, Suspensionen, leicht rekonstituierbare Trockenzubereitungen sowie Sprays. Eine weitere Möglichkeit sind Suppositorien für die Anwendung im Rektum. Die Anwendung in einem Depot in gelöster Form, einer Trägerfolie oder einem Pflaster, gegebenenfalls unter Zusatz von die Hautpenetration fördernden Mitteln, sind Beispiele für geeignete perkutane Applikationsformen. Beispiele für Hilfs- und Zusatzmitteln für die oralen Applikationsformen sind Sprengmittel, Gleitmittel, Binder, Füllmittel, Formtrennmittel, gegebenenfalls Lösungsmittel, Geschmacksstoffe, Zucker, insbesondere Trägermittel, Verdünnungsmittel, Farbstoffe, Antioxidantien etc. Für Suppositorien können u.a. Wachse bzw. Fettsaureester und für parenterale Applikationsmittel Trägerstoffe, Konservierungsmittel, Suspensionshilfsmittel etc. verwendet werden. Die an Patienten zu verabreichenden Wirkstoffmengen variieren in Abhängigkeit vom Gewicht des Patienten, von der Applikationsart und dem Schweregrad der Erkrankung. Aus oral, rektal oder perkutan anwendbaren Zubereitungsformen kann die erfindungsgemäße Verbindung verzögert freigesetzt werden. Bei der erfindungsgemäßen Indikation sind entsprechende Retard-Formulierungen, insbesondere in Form eines "Once-daily"-Präparats, das nur einmal am Tag eingenommen werden muß, besonders bevorzugt.

Bevorzugt sind Arzneimittel, die wenigstens 0,05 bis 90,0 % des Wirkstoffes enthalten, insbesondere niedrige wirksame Dosierungen, um Nebenwirkungen zu vermeiden, Erfindungsgemäß erfolgt die Applikation von 0,01 - 5 mg/kg, vorzugsweise 0,03 bis 2 mg/kg, insbesondere 0,05 bis 1 mg/kg Körpergewicht der erfindungsgemäßen Verbindung.

Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylen-fettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid. Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Herstellung dieser Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels mit einem pharmazeutischen Träger, z.B. herkömmlichen Tabletteninhaltsstoffen, wie Maisstärke, Lactose, Saccharose, Sorbitol, Talkum, Magnesiumstearat. Dicaiciumphosphat oder pharmazeutisch akzeptable Gummis, und pharmazeutischen Verdünnungsmitteln, wie z.B. Wasser, granuliert werden, um eine feste Zusammensetzungzu bilden, die Wirkstoff in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Auch wenn die erfindungsgemäß hergestellten Arzneimittel lediglich geringe Nebenwirkungen zeigen, kann es beispielsweise zur Vermeidung von bestimmten Formen der Abhängigkeit von Vorteil sein, neben der genannten erfindungsgemäßen Verbindung auch Morphinantagonisten, insbesondere Naloxon, Naltrexon und/oder Levallorphan, zu verwenden. In Untersuchungen wurde beispielsweise für Morphin und Verbindung 10 (s. Beispiel 0 folgend) nachgewisen, daß die Substanzen auch mit Naloxon im neuropathischem Schmerz wirksam sind.

Die folgenden Beispiele sollen die Erfindung erläutern, ohne daß der Gegenstand der Erfindung darauf beschränkt wäre.

### Beispiele

### Beispiel 0: Getestete Substanzen:

Die folgenden Verbindungen wurden getestetet und werden im folgenden entsprechend Tabelle 1 als Verbindung (bzw. Verb.) 1 etc. abgekürzt:

**Tabelle 1:**

| **Name:** | **Verbindung** |
|---|---|
| (2RS,3RS)-1-Dimethylamino-3-(3-methoxy-phenyl)-2-methyl-pentan-3-ol: Hydrochlorid | **Ref. 1** |
| (2S,3S)-1-Dimethylamino-3-(3-methoxy-phenyl)-2-methyl-pentan-3-ol; Hydrochlorid | **Ref. 2** |
| (2R,3R)-1-Dimethylamino-3-(3-methoxy-phenyl)-2-methyl-pentan-3-ol; Hydrochlorid | **Ref. 3** |
| | |
| (1RS,2RS)-3-(3-Dimethylamino-1-hydroxy-1,2-dimethyl-propyl)-phenol; Hydrochlorid | **Ref. 5** |
| (1S,2S)-3-(3-Dimethylamino-1-hydroxy-1,2-dimethyl-propyl)phenol; Hydrochlorid | **Ref. 6** |
| (1R,2R)-3-(3-Dimethylamino-1-hydroxy-1,2-dimethyl-propyl)phenol; Hydrochlorid | **Ref. 7** |
| (2RS,3RS)-3-(Difluoromethyl-phenyl)-1-dimethylamino-2-methyl-pentan-3-ol; Hydrochlorid | **Ref. 8** |
| (1R,2R)-3-(3-Dimethylamino-1-ethyl-2-methyl-propyl)-phenol; Hydrochlorid | **9** |
| (1S,2S)-3-(3-Dimethylamino-1-ethyl-2-methyl-propyl)phenol; Hydrochlorid | **Ref. 10** |
| | |
| | |
| | |
| 3-(3-Dimethylamino-1,2-dimethyl-propenyl)-phenol; Hydrochlorid | **Ref. 14** |
| | |

| **Zusätzlich:** | |
|---|---|
| Morphin | **Mor** |
| Gabapentin | **GBP** |

| | |
|---|---|
| Ref. = Referenz | |

### Beispiel 1. Bennett

### Neuropathischer Schmerz an der Ratte

Die Untersuchung auf Wirksamkeit im neuropathischen Schmerz wurde im Bennett-Modell (chronic constriction injury; Bennett und Xie, 1988, Pain 33: 87-107) untersucht.

Sprague-Dawley Ratten mit einem Gewicht von 140-160g werden unter Nembutal-Narkose mit vier losen Ligaturen des rechten nervus ischiaticus versehen. Die Tiere entwickeln an der vom geschädigten Nerv innervierten Pfote eine Überempfindlichkeit, die nach einer Erholungsphase von einer Woche über etwa vier Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen. Bezogen auf den Vorwert vor Substanzapplikation wird die Substanzwirkung über einen Zeitraum von einer Stunde an vier Zeitpunkten (15, 30, 45, 60 min. nach Applikation) bestimmt und die resultierend Fläche unter der Kurve (AUD) sowie die Hemmung der Kälte-Allodynie zu den einzelnen Meßpunkten in Prozent Wirkung zur Vehikelkontrolle (AUD) bzw. zum Ausgangswert (Einzelmeßpunkte) ausgedrückt. Die Gruppengröße beträgt n=10.

Die Ergebnisse sind mit denen aus Beispiel 2 in Tabelle 2 (s.u.) zusammengefaßt.

### Beispiel 2: Chung

### In vivo Experimente nach Chung

In mänlichen Sprague-Dawley-Ratten wurden an den linken L5/L6 SpinalNerven Spinal-Nerven-Ligaturen gemäß Kim und Chung (1992 Pain 50, 355-363.) angelegt. 4 bis 6 Tage nach der Operation wurde die taktile Schwellen-Basislinie (Rückzugs-Schwellen/withdrawal threshholds) an der ipsi- und contralateralen Hinterpfote durch ein elektronisches vonFrey-Anethesiometer (IITC Life Science, USA) gemessen. Nach dem Test und Messung der Basislinie wurden Morphin, Gabapentin und einige der oben genannten erfindungsgemäß verwendeten Verbindungen gegeben. Die taktilen Rückzugs-Schwellen (withdrawal threshholds) wurden 30 Minuten nach der Gabe gemessen. Die Ergebnisse sind als ED50 bzw. % maximal possible effect (%MPE; % des maximal möglichen Effekts) auf der ipsilateralen Seite angegeben, in dem man die Basisline als 0% und die Rückzugs-Schwelle einer Kontroll-Gruppe als 100%MPE annimmt.

Die Ergebnisse sind mit denen aus Beispiel 1 in Tabelle 2 zusammengefaßt:

**Tabelle 2: Prüfung der Hemmung im neuropathischen Schmerz an der Ratte nach intraperitonealer (i.p.) oder peroraler (p.o.) Substanzgabe**

| **Verbindung** | **Kälte Allodynie ED50 mg/kg i.p. (95% VB) [Bandbreite der Meßwerte]** | | **Taktile Allodynie ED50 mg/kg i.p. (95% VB) [Bandbreite der Meßwerte]** |
|---|---|---|---|
| **Morphin** | 7,1 [5,7-9,4] | | 4,6 [3,8-5,7] |
| **Gabapentin** | 214 (p.o.) | | 92,6 |
| **Ref. 1**. | 11,1 [9,9-12,3] | | 10.1 |
| **Ref. 2** | 26,0 | | - |
| **Ref. 3** | 9,2 | | - |
| | | | |
| **Ref. 5** | 15,0 | | - |
| **Ref. 6** | 32,6 | | 4,65 |
| **Ref. 7** | 11,1 | | - |
| **Ref. 8** | 17,1 | | - |
| **9** | 13 | | 8,15 [3,8-14,5] |
| **Ref. 10** | 1,2 [0,6-3,1] | | 1,5 [0,74-12,54] |
| | | | |
| | | | |
| | | | |
| **14** | 3,6 | | - |
| | | | |

Die Verbindungen zeigen alle eine deutliche und dosisabhängige Hemmung von Kälte-Allodynie in Bennett Tieren und von taktiler Allodynie in Chung Tieren. Darüber hinaus ist in Chung Tieren teilweise eine erstaunlich lange Wirkdauer von bis zu 30 Stunden nach ip-Applikation zu beobachten.

### Beispiel 3: in vivo Experimente in Ratten mit diabetischer Neuropathie

Männliche Sprague Dawley Ratten mit einem Körpergewicht von 160-180g erhielten eine intraperitoneale Injektion von Streptozotozin (75mg/kg Körpergewicht) welches in Citratpuffer pH 4.6 gelöst war. Eine Woche später wurden diabetische Tiere über Messung des Blutglukosespiegels identifiziert und bei einem Blutglukosespiegel von ≥ 17mM in die Studie eingeschlossen. Drei und vier Wochen nach Streptozotozingabe wurde in diabetischen Tieren die mechanische Reaktionsschwelle nach der Methode von Randall und Sellito (1957 Arch. Int. Pharmacodyn. 61: 409-419) vor (Vortest) und zu verschiedenen Zeitpunkten nach Gabe von Testsubstanz oder Vehikel (Nachtest) bestimmt Diabetische Tiere zeigen im Vergleich zu Kontrolltieren, die anstelle des Streptozotozins zum gleichen Zeitpunkt Vehikellösung erhielten eine erniedrigte mechanische Reaktionsschwelle und somit eine mechanische Hyperalgesie. Der maximal auf die Hinterpfote ausgeübte Druck betrug 250g. Der Endpunkt der mechanischen Reaktionsschwelle in Gramm wurde anhand der Reaktion des Tieres (Wegziehen der Hinterpfote, Vokalisation oder Fluchtreaktion) bestimmt. Die Gruppengröße in den einzelnen Dosisstufen betrug n=10. Die maximal mögliche Antwort in Prozent (%MPE = % des maximal möglichen Effektes) wurde berechnet nach der Formel %MPE= (Nachtest- Vortest) / (250-Vortest) x 100. ED50 Werte (Dosis bei der halbmaximale Hemmung auftrat) wurden über Regressionsanalyse aus den %MPE Werten zum Zeitpunkt der maximalen Wirkung bestimmt.

Tabelle 3: Prüfung der Hemmung im diabetischen neuropathischen Schmerz an der Ratte nach intraperitonealer (i.p.) Substanzgabe

| **Verbindung** | **Mechanische Hyperalgesia ED50 mg/kg i.p. (95% Vertrauensbereich)** | **Mechanische Hyperalgesia Maximaleffekt in %MPE bei (Dosis mg/kg i.p.)** |
|---|---|---|
| **Morphin** | 3.0 (1.8-4.0) | 89% (10mg/kg) |
| **Gabapentin** | 225 (186-274) | 80% (464mg/kg) |
| **Tramadol** | 9.2 (7.0-11.8) | 86% (21.5mg/kg) |
| **Verbindung 9** | 8.9 (7.1-11.1) | 100% (31.6mg/kg) |

Verbindung **9** erreichte unter den getesteten Verbindungen den höchsten Maximaleffekt.

### Beispiel 4: Toleranzen gegen Morphin

Die zugrundeliegende Fragestellung ist die Wirkung einer Testsubstanz mit vermutet opioidem Wirkmechanismus in Patienten, die tolerant oder therapieresistent gegenüber Morphin sind. In Bennett Tieren (gemäß Beispiel 1), die gegenüber Morphin eine Toleranz entwickelt haben, zeigten die getesteten Verbindungen noch eine deutliche anti-allodynische Wirkung. In der Tabelle 4 sind die Wirkung von Testsubstanzen in naiven (nicht Morphin toleranten) Tieren und Morphin toleranten Tieren gegenübergestellt. Morphin (Mor) zeigt verständlicherweise keine Wirkung mehr, während die anderen getesteten Verbindungen eine deutliche Hemmung der Kälte-Allodynie in diesen Tieren bewirken. Getestet wurden analog zu Beispiel 1 Morphin [10 mg/kg i.p.], Verbindung 9 [10 und 21,5 mg/kg i.p.], Verbindung 10 [0,46 und 1 mg/kg i.p.], Verbindung 4 [21,5 mg/kg i.p.] und Verbindung 11 [21,5 mg/kg i.p.].

Tabelle 4: Prüfung der Hemmung im neuropathischen Schmerz an der Ratte nach intraperitonealer (i.p.) Substanzgabe in naiven und Morphin toleranten Tieren in %AUD.

| Verbindung [Dosis mg/kg i.p.] | Kälte Allodynie (%AUD) Morphin tolerante Tiere | Kälte Allodynie (%AUD) naive Tiere |
|---|---|---|
| Morphin [10] | -22,0 | 78,6 |
| 9 [10] | 37,3 | 49,3 |
| 9 [21,5] | 36,7 | 53,4 |
| 10 [0,46] | 22,9 | 29,0 |
| 10 [1] | 39,9 | 42,7 |
| 1 [2,15] | 13,0 | 59,2 |
| 4 [21,5] | 75,2 | 65,5 |
| 11 [21,5] | 42,6 | 69,2 |

### Beispiel 5: Parenterale Applikationsform

20 g Verbindung 9 wird in 1 l Wasser für Injektionszwecke bei Raumtemperatur gelöst und anschließend durch Zugabe von NaCl auf isotone Bedingungen eingestellt.

### Beispiel 6: Vergleich Mono- und Polyneurpathischen Schmerz

### Experimentelle Durchführung

Männliche Sprague Dawley Ratten (140-180g, Janvier, Frankreich) werden bei Standardbedingungen (6.00-18.00 Uhr Licht, 18.00-6.00 Uhr Dunkelheit; 20-24°C Raumtemperatur; 35-70% relative Luftfeuchte; Leitungswasser und Standardfutter ad libitum) in Gruppen zu fünf Tieren in Macrolon Typ 4 Käfigen gehalten.

### Mononeuropathie (Spinale Nervenligatur, SNL) Referenzbeispiel

Unter Pentobarbitalnarkose (Narcoren, 60mg/kg i.p., Merial GmbH, Deutschland) werden unilateral an der linken Seite die Spinalnerven L5 und L6 mit einem Seidenfaden (NC-silk black, USP 5/0, metric 1, Braun Melsungen AG, Deutschland) fest abgebunden (Kim und Chung, Pain 1992; 50: 355-63). Nach der Operation können sich die Tiere eine Woche erholen und entwickeln innerhalb dieser Zeit eine Überempfindlichkeit an der ipsilateralen (linken) Pfote. Die Überempfindlichkeit gegen einen Druckreiz kann mit Hilfe eines elektronischen von Frey Filaments (Somedic, Schweden) gemessen werden. Dazu werden die Tiere auf ein Gitter unter eine Haube gesetzt. Nach Habituation an die Umgebung werden geschädigte (ipsilaterale, linke) und ungeschädigte (kontralaterale, rechte) Hinterpfote solange ansteigendem Druck auf die Pfotenunterseite ausgesetzt, bis die entsprechende Pfote weggezogen wird. Der Median aus fünf Versuchen bestimmt die Wegziehschwelle eines Testzeitpunktes. Die Tiere werden an beiden Hinterpfoten vor und zu verschiedenen Zeiten nach Substanz- oder Vehikelgabe getestet. Für jedes Tier wird die Differenz zwischen Testwert und Vortest für ipsi- und kontralaterale Seite bestimmt und für die Gruppen, die sich aus jeweils zehn Tieren zusammensetzen das Ergebnis als Mittelwert (MW) und Standardfehler des Mittelwertes (SEM) ausgedrückt. Der Unterschied zwischen den mittleren Differenzwerten der ipsi- und kontralateralen Seite definiert die Mononeuropathie induzierte Überempfindlichkeit. Die Signifikanz einer Substanzwirkung wird auf Basis der Differenzwerte gegen die Vehikelgruppe für die ipsi- und kontralaterale Seite mittels zweifaktorieller Varianzanalyse und posthoc Analyse nach Bonferroni bestimmt.

### Polyneuropathie (Streptozotozin induzierte diabetische Neuropathie, STZ)

Ratten erhalten eine einmalige i.p. Gabe von Streptozotozin (STZ, Sigma Aldrich Chemie, Deutschland) oder Vehikel (0.1 mM Zitratpuffer, pH 4.6). Nach einer Woche werden die Blutzuckerwerte bestimmt und STZ behandelte Tiere, die eine Blutzuckerwert von ≥ 17 mM aufweisen als diabetisch in den Versuch eingeschlossen. Diabetische Tiere entwickeln eine Überempfindlichkeit an den Hinterpfoten. Die Überempfindlichkeit gegen einen Druckreiz kann mit Hilfe eines Druckschmerzgerätes (Algesiometer; Ugo Basile, Italien) nach der Methode von Randall und Selitto (Arch. Int. Pharmcodyn. 1957; 111: 409-19) in diabetischen im Vergleich zu gesunden Kontrolltieren gleichen Gewichts in der dritten Wochen nach STZ Behandlung bestimmt werden. Nach Habituation an die Umgebung wird die rechte Hinterpfote von geschädigten (diabetisch) und ungeschädigten (gesund) Tieren solange ansteigendem Druck auf die Pfotenoberseite ausgesetzt, bis die entsprechende Pfote weggezogen wird, oder das Tier vokalisiert. Dieser Wert bestimmt die Wegziehschwelle eines Testzeitpunktes. Diabetische und gesunde Tiere werden vor und zu verschiedenen Zeiten nach Substanz- oder Vehikelgabe getestet. Für jedes Tier wird die Differenz zwischen Testwert und Vortest bestimmt und für die Gruppen, die sich aus jeweils zehn Tieren zusammensetzen das Ergebnis als Mittelwert (MW) und Standardfehler des Mittelwertes (SEM) ausgedrückt. Der Unterschied zwischen den mittleren Differenzwerten der diabetischen und gesunden Tiere definiert die Polyneuropathie induzierte Überempfindlichkeit. Die Signifikanz einer Substanzwirkung wird auf Basis der Differenzwerte gegen die Vehikelgruppe für diabetische und gesunde Tiere mittels zweifaktorieller Varianzanalyse und posthoc Analyse nach Bonferroni bestimmt.

### Ergebnisse

### Mononeuropathischer Schmerz

(1R, 2R)-3-(3-dimethylamino-1-ethyl-2-methyl-propyl)-phenol hydrochlorid 9 (0,1 - 10 mg/kg, i.v., Tab.1) zeigt eine dosisabhängige Erhöhung der Wegziehschwelle an der ipsilateralen Hinterpfote. Die minimale effektive Dosis bei der statistische Signifikanz erreicht wird liegt bei 1 mg/kg. Der mittlere Differenzwert zwischen der ipsilateralen Wegziehschwelle gesunder Kontrolltiere und der ipsilateralen Wegziehschwelle mononeuropathischer Tiere beträgt in dieser Versuchsreihe 36 g. Volle Hemmung der mononeuropathisch induzierten Wegziehschwellenreduktion wird somit bei Werten (Testwert - Vorwert) von ≥ 36 g an der ipsilateralen Seite erreicht. Zeitpunkte, an denen dieser Wert erreicht oder überschritten wird sind in der Tabelle mit Rahmen und fett hinterlegt. Kontralaterale Messwerte sind nicht in diese Betrachtung eingeschlossen. In der höchsten Dosisgruppe von 10 mg/kg i.v. wird bei 30 min volle Hemmung der mononeuropathisch induzierten Wegziehschwellenreduktion erreicht. Auch die kontralaterale Wegziehschwelle wird dosisabhängig erhöht. Die minimale effektive Dosis bei der statistische Signifikanz erreicht wird liegt bei 10 mg/kg.

**Tab.1: (1R, 2R)-3-(3-dimethylamino-1-ethyl-2-methyl-propyl)-phenol hydrochlorid (9) Mononeuropathie (Testwert - Vortest (g); * p < 0.05 gegen Vehikel; n.s. nicht signifikant gegen Vehikel)**

| **Dosis (mg/kg, i.v.)** | **Seite** | | **30 min** | **60 min** | **180 min** |
|---|---|---|---|---|---|
| Vehikel | Ipsi | MW | 0,70 | 0,06 | -1,33 |
| | | SEM | 1,16 | 1,31 | 1,09 |
| | | Signifikanz | n.s. | n.s. | n.s. |
| | Contra | MW | -4,06 | 0,83 | -3,79 |
| | | SEM | 2,63 | 3,77 | 2,90 |
| | | Signifikanz | n.s. | n.s. | n.s. |
| 0,1 | Ipsi | MW | 1,74 | -0,59 | -2,24 |
| | | SEM | 1,55 | 1,44 | 1,15 |
| | | Signifikanz | n.s. | n.s. | n.s. |
| | Contra | MW | 0,06 | -3,13 | -4,30 |
| | | SEM | 4,52 | 4,12 | 3,01 |
| | | Signifikanz | n.s. | n.s. | n.s. |
| 0,316 | Ipsi | MW | 5,78 | 2,21 | 1,68 |
| | | SEM | 2,34 | 2,93 | 1,41 |
| | | Signifikanz | n.s. | n.s. | n.s. |
| | Contra | MW | 0,39 | -6,20 | 2,21 |
| | | SEM | 4,71 | 3,03 | 3,81 |
| | | Signifikanz | n.s. | n.s. | n.s. |
| 1 | Ipsi | MW | 12,86 | 7,09 | 2,01 |
| | | SEM | 2,42 | 0,80 | 0,64 |
| | | Signifikanz | * | n.s. | n.s. |
| | Contra | MW | 2,90 | 2,45 | -6,65 |
| | | SEM | 3,32 | 3,92 | 2,45 |
| | | Signifikanz | n.s. | n.s. | n.s. |
| 3,16 | Ipsi | MW | 18,43 | 20,32 | 11,06 |
| | | SEM | 2,90 | 2,39 | 1,45 |
| | | Signifikanz | * | * | * |
| | Contra | MW | 8,02 | 2,85 | -0,34 |
| | | SEM | 4,13 | 3,09 | 3,81 |
| | | Signifikanz | n.s. | n.s. | n.s. |
| 10 | Ipsi | MW | **42,05** | 19,47 | 13,65 |
| | | SEM | 3,50 | 1,52 | 1,40 |
| | | Signifikanz | * | * | * |
| | Contra | MW | 31,85 | 10,62 | 4,51 |
| | | SEM | 3,23 | 4,92 | 4,17 |
| | | Signifikanz | * | n.s. | n.s. |

### Polyneuropathischer Schmerz

(1R, 2R)-3-(3-dimethylamino-1-ethyl-2-methyl-propyl)-phenol hydrochlorid 9 (0,1 -1 mg/kg, i.v., Tab.2) zeigt eine dosisabhängige Erhöhung der Wegziehschwelle an der Hinterpfote diabetischer Ratten. Die minimale effektive Dosis bei der statistische Signifikanz erreicht wird liegt bei 0,3 mg/kg. Der mittlere Differenzwert zwischen der Wegziehschwelle von naiven Kontrolltieren und der Wegziehschwelle polyneuropathischer Tiere beträgt in dieser Versuchsreihe 43 g. Volle Hemmung der polyneuropathisch induzierten Wegziehschwellenreduktion wird somit bei Werten (Testwert - Vorwert) von ≥ 43 g in diabetischen Tieren erreicht. Zeitpunkte, an denen dieser Wert erreicht oder überschritten wird sind in der Tabelle grau hinterlegt. Messwerte von naiven Tieren sind nicht in diese Betrachtung eingeschlossen. In der höchsten Dosisgruppe von 1 mg/kg i.v. wird bei 15 und 30 min volle Hemmung der polyneuropathisch induzierten Wegziehschwellenreduktion erreicht.

Auch die Wegziehschwelle von gesunden Kontrolltieren wird dosisabhängig erhöht. Die minimale effektive Dosis bei der statistische Signifikanz erreicht wird liegt bei 1 mg/kg.

**Tab.2: (1R, 2R)-3-(3-dimethylamino-1-ethyl-2-methyl-propyl)-phenol hydrochlorid (9) Polyneuropathie (Testwert - Vortest (g); *p < 0.05 gegen Vehikel; n.s. nicht signifikant gegen Vehikel)**

| **Dosis (mg/kg, i.v.)** | **Gruppe** | | **15 min** | **30 min** | **45 min** | **60 min** |
|---|---|---|---|---|---|---|
| 0,1 | Diabetes | MW | 1,0 | -2,0 | 6,0 | -2,0 |
| | | SEM | 6,4 | 5,5 | 3,4 | 5,5 |
| Vehikel | | MW | 3,0 | 2,0 | -11,0 | 0,0 |
| | | SEM | 4,2 | 3,6 | 3,5 | 3,7 |
| | | Signifikanz | n.s. | n.s. | n.s. | n.s. |
| 0,1 | Naiv | MW | 4,0 | -12,0 | -2,0 | -5,0 |
| | | SEM | 4,8 | 7,7 | 5,3 | 6,2 |
| Vehikel | | MW | -9,0 | -2,0 | -5,0 | -3.0 |
| | | SEM | 4,8 | 5,1 | 4,5 | 2,1 |
| | | Signifikanz | n.s. | n.s. | n.s. | n.s. |

| **Dosis (mg/kg, i.v.)** | | | **15 min** | **30 min** | **45 min** | **60 min** |
|---|---|---|---|---|---|---|
| 0,316 | Diabetes | MW | 35,0 | 30,0 | 24,0 | 16,0 |
| | | SEM | 9,2 | 6,3 | 6,9 | 4,8 |
| Vehikel | | MW | -6,0 | -5,0 | 0,0 | 1,0 |
| | | SEM | 3,7 | 6,7 | 6,0 | 3,5 |
| | | Signifikanz | * | * | * | n.s. |
| 0,316 | Naiv | MW | 17,0 | -5,0 | -5,0 | 2,0 |
| | | SEM | 3,3 | 7,3 | 4,5 | 2,5 |
| Vehikel | | MW | -6,0 | -5,0 | -14,0 | 2,0 |
| | | SEM | 6,7 | 5,8 | 6,7 | 4,4 |
| | | Signifikanz | n.s. | n.s. | n.s. | n.s. |

| **Dosis (mg/kg, i.v.)** | **Seite** | | **15 min** | **30 min** | **45 min** | **60 min** |
|---|---|---|---|---|---|---|
| 1 | Diabetes | MW | **54,0** | **49,0** | 34,0 | 27,0 |
| | | SEM | 10,8 | 8,7 | 6,5 | 6,2 |
| Vehikel | | MW | 1,0 | -10,0 | 4,0 | 4,0 |
| | | SEM | 5,9 | 3,3 | 6,4 | 3,4 |
| | | Signifikanz | * | * | * | * |
| 1 | Naiv | MW | 42,0 | 28,0 | -2,0 | 7,0 |
| | | SEM | 7,6 | 7,4 | 6,3 | 4,5 |
| Vehikel | | MW | -4,0 | -5,0 | -15,0 | -2,0 |
| | | SEM | 5,6 | 8,7 | 3,1 | 3,6 |
| | | Signifikanz | * | * | n.s. | n.s. |

(1R, 2R)-3-(3-dimethylamino-1-ethyl-2-methyl-propyl)-phenol hydrochlorid 9 bewirkt in mono- und polyneuropathischem Schmerz eine dosisabhängige Erhöhung der druckvermittelten Wegziehschwelle. In beiden Schmerzmodellen bewirkt (1R, 2R)-3-(3-dimethylamino-1-ethyl-2-methyl-propyl)-phenol eine selektive Hemmung der pathologisch induzierten Schmerzantwort, ohne gleichzeitig die normale Schmerzantwort zu beeinflussen. Ein klarer Unterschied tritt in der Wirkstärke von (1R, 2R)-3-(3-dimethylamino-1-ethyl-2-methyl-propyl)-phenol in beiden Modellen auf. Während im polyneuropathischen Schmerzmodell bereits eine signifikante Hemmung bei 0.316 mg/kg i.v. nachweisbar ist, tritt eine signifikante Hemmung im mononeuropathischen Schmerzmodell erst bei 1 mg/kg i.v. auf, also bei einer dreifach höheren Dosierung. Ähnlich verhält es sich mit der Dosis, bei der der maximale Effekt erreicht wird. Im polyneuropathischen Schmerzmodell wird die vollständige Hemmung bei 1 mg/kg i.v. erreicht, während sie im mononeuropathischen Schmerzmodell erst bei 10 mg/kg i.v., also in einer zehnfach höheren Dosis erreicht wird. Diese Daten zeigen, dass (1R, 2R)-3-(3-dimethylamino-1-ethyl-2-methyl-propyl)-phenol für die Behandlung von polyneuropathischen Schmerzzuständen in besonderer Weise geeignet ist.

## Patentansprüche

1. (1R,2R)-3-(3-Dimethylamino-1-ethyl-2-methylpropyl)-phenol in dargestellter Form oder in Form eines physiologisch verträglichen Salzes, zur Anwendung bei der Behandlung von diabetischem neuropathischem Schmerz oder diabetischer Neuropathie durch Applikation von 0,01 bis 5 mg/kg Körpergewicht.

2. (1R,2R)-3-(3-Dimethylamino-1-ethyl-2-methylpropyl)-phenol zur Anwendung nach Anspruch 1, wobei der diabetische neuropathische Schmerz oder die diabetische Neuropathie peripher ist.

## Claims

1. (1R,2R)-3-(3-Dimethylamino-1-ethyl-2-methylpropyl)-phenol in the form shown or in the form of a physiologically acceptable salt, for use in the treatment of diabetic neuropathic pain or diabetic neuropathy by administration of from 0.01 to 5 mg/kg of body weight.

2. (1R,2R)-3-(3-Dimethylamino-1-ethyl-2-methylpropyl) - phenol for use according to claim 1, wherein the diabetic neuropathic pain or the diabetic neuropathy is peripheral.

## Revendications

1. (1R,2R)-3-(3-diméthylamino-1-éthyl-2-méthylpro-pyl)-phénol sous forme préparée ou sous la forme d'un sel physiologiquement acceptable, pour utilisation dans le traitement de la douleur neuropathique diabétique ou de la neuropathie diabétique par l'administration de 0,01 à 5 mg/kg de poids corporel.

2. (1R,2R)-3-(3-diméthylamino-1-éthyl-2-méthylpro-pyl)-phénol pour une utilisation selon la revendication 1, la douleur neuropathique diabétique ou la neuropathie diabétique étant périphérique.
